# EUROPEAN PATENT SPECIFICATION

(11) **EP 0 789 563 B1**
(45) Date of publication and mention of the grant of the patent: **16.06.2004**
(21) Application number: 95938824.0
(22) Date of filing: 05.10.1995
(51) Int. Cl.: A61K 31/00, A61K 35/00, A61K 39/145, C12N 7/00, C12N 7/01, C07K 14/02, C07K 14/18, A61P 31/14

(54) **HEPATITIS VIRUS B AND C VACCINES**
IMPFSTOFFE GEGEN DEN HEPATITIS B UND C VIRUS
VACCINS CONTRE LE VIRUS DE L'HEPATITE B ET C

(30) Priority: 05.10.1994 US 318248; 06.06.1995 US 467859
(43) Date of publication of application: 20.08.1997
(73) Proprietor: APOLLON, INC., Malvern, PA 19355-1423 (US); THE MASSACHUSETTS GENERAL HOSPITAL, Boston Massachusetts 02114 (US)
(72) Inventor: PACHUK, Catherine J., Lansdowne, PA 19050 (US); WANDS, Jack, Waban, MA 02168 (US); WAKITA, Takaji, Tokyo 175 (JP); ZURAWSKI, Vincent R., Jr., Westtown, PA 19393 (US); CONEY, Leslie R., Rosemont, PA 19010 (US); TOKUSHIGE, Katsutoshi, Boston, MA 02114 (US)
(74) Representative: Stebbing, Peter John Hunter
(86) International application number: PCT/US1995/013552
(87) International publication number: WO 1996/010997

(56) References cited:
- WO-A-93/15193
- WO-A-93/15207
- CA-A- 2 055 149
- JP-A- 6 092 996
- JP-A- 6 141 870
- DATABASE WPI Section Ch, Week 9444 Derwent Publications Ltd., London, GB; Class B04, AN 94-354772 XP002072536 & JP 06 279 500 A (IMMUNO JAPAN KK)
- MAJOR M E ET AL: "DNA-based immunization with chimeric vectors for the induction of immune responses against the hepatitis C virus nucleocapsid" JOURNAL OF VIROLOGY., vol. 69, no. 9, September 1995, US, pages 5798-5805, XP002072535
- JOURNAL OF VIROLOGY, Volume 67, Number 10, issued October 1993, YOSHIKAWA et al., "Chimeric Hepatitis B Virus Core Particles With Parts or Copies of the Hepatitis C Virus Core Protein", pages 6064-6070.
- PROC. NATL. ACAD. SCI. U.S.A., Volume 83, issued December 1986, ITOH et al., "A Synthetic Peptide Vaccine Involving the Product of the Pre-S(2) Region of Hepatitis B Virus DNA: Protective Efficacy in Chimpanzees", pages 9174-9178.
- PROC. NATL. ACAD. SCI. U.S.A., Volume 88, issued March 1991, HAN et al., "Characterization of the Terminal Regions of Hepatitis C Viral RNA: Identification of Conserved Sequences in the 5' Untranslated Region and Poly (A) Tails at the 3' End", pages 1711-1715.
- FEBS, Volume 332, Number 1,2, issued October 1993, WELLS, "Improved Gene Transfer by Direct Plasmid Injection Associated With Regeneration in Mouse Skeletal Muscle", pages 179-182.
- JOURNAL OF MEDICAL VIROLOGY, Volume 40, issued 1993, WANG et al., "Prevalence, Genotypes and an Isolate (HC-C2) of Hepatitis C Virus in Chinese Patients With Liver Disease", pages 254-260.
- MICHEL M. ET AL: 'Induction of anti-human immunodeficiency virus (HIV) neutralizing antibodies in rabbits immunized with recombinant HIV-hepatitis B surface antigen particles' PNAS vol. 85, 1998, pages 7957 - 7961
- DELPEYROUX F. ET AL: 'A poliovirus neutralization epitope expressed on hybrid hepatitis B surface antigen particles' SCIENCE vol. 233, 1986, pages 472 - 475

## Description

### FIELD OF THE INVENTION

The present invention relates to gene constructs which are useful as anti-hepatitis C virus vaccine components in genetic immunization protocols, in protecting individuals against hepatitis C virus infection, treating individuals suffering from hepatitis C virus infection; to recombinant chimeric gene constructs which are useful as anti-hepatitis B virus and/or anti-hepatitis C virus vaccine components in genetic immunization protocols, in protecting individuals against hepatitis B virus and/or hepatitis C virus infection and treating individuals suffering from hepatitis B virus and/or hepatitis C virus infection.

### BACKGROUND OF THE INVENTION

Hepatitis C virus (HCV), the major etiologic agent of transfusion acquired non-A, non-B hepatitis, is responsible for approximately 150,000 new cases of acute viral hepatitis annually in the United States. Greenberger, N.J., 1983, "New Approaches for Hepatitis C", *Contemporary Internal Medicine* **Feb**:64. Approximately half of these infections progress to a chronic infection that can be associated with cirrhosis and/or hepatocellular carcinoma (Alter, *et al.*, *Science,* **1992,** *258,* 135-140; and Alter, *et al., New Eng. J. Med.,* **1992,** *327,* 1899-1905). In addition, HCV infection is an independent risk factor for the development of hepatocellular carcinoma as shown by the prevalence of anti-HCV antibodies (Colombo, et al., *Lancet*, **1989,** *ii*, 1006-1008; Saito, *et al., Proc. Natl. Acad. Sci. USA,* **1990,** *87,* 6547-6549; Simonetti, *et al.*, An. *Int. Med.,* **1992,** *116,* 97-102; and Tsukuma, *et al., New Eng. J. Med.,* 1993, 328, 1797-1801).

HCV is an enveloped, positive stranded RNA virus, approximately 9,500 nucleotides in length, which has recently been classified as a separate genus within the Flavivirus family (Heinz, F.X., *Arch. Virol. (Suppl.),* **1992,** *4,* 163-171) . Different isolates show considerable nucleotide sequence diversity leading to the subdivision of HCV genomes into at least eight genotypes (Simmonds, *et al.*, *J. Gen. Virol.,* **1993,** *74,* 2391-2399). In all genotypes, the viral genome contains a large open reading frame (ORF) that encodes a precursor polyprotein of 3010 to 3033 amino acids of approximately 330 Kd (Choo, *et al., Proc. Natl. Acad. Sci. USA,* **1991,** *88,* 2451-2455; Inchauspe, *et al.*, *Proc. Natl. Acad. Sci. USA,* **1991,** *88,* 10292-10296; Kato, *et al., Proc. Natl. Acad. Sci. USA,* **1990,** *87,* 9524-9528; Okamoto, *et al., J. Gen. Virol.,* **1991,** *72,* 2697-2704; and Takamizawa, *et al., J. Gen. Virol.,* **1991,** *65,* 1105-1113) .

Individual HCV polypeptides are produced by proteolytic processing of the precursor polypeptide to produce core (C), envelope (E1, E2) and non-structural (NS2-NS5) proteins (Bartenschlager, *et al., J. Gen. Virol.,* **1993,** *67,* 3835-3844; Grakoui, *et al.*, *J. Gen. Virol.,* **1993,** *67,* 2832-2843; and Selby, *et al., J. Gen. Virol.,* **1993,** *74,* 1103-1113). This proteolysis is catalyzed by a combination of both cellular and viral encoded proteases.

In addition to the translated region, the HCV genome also contains both a 5' untranslated region (5' UTR) and a 3' untranslated region (3' UTR). The 5' UTR of 324 to 341 nucleotides represents the most highly conserved sequence among all HCV isolates reported to date (Han, *et al.*, *Proc. Natl. Acad. Sci. USA,* **1991,** *88,* 1711-1715; and Bukh, *et al., Proc. Natl. Acad. Sci. USA,* **1992,** *89,* 4942-4946). This 5' UTR has been postulated to contain important regulatory elements for replication and/or translation of HCV RNAs. The 5' UTR also contains several small open reading frames (ORF) but there is presently no evidence to suggest that these ORF sequences are actually translated.

The HCV core gene may be an important target for nucleic acid-based antiviral approaches. The first 191 amino acids of the HCV polyprotein precursor are believed to represent the viral nucleocapsid protein. This protein is comprised of a basic, RNA-binding amino-terminal domain and a highly hydrophobic carboxy-terminal region (Bukh, *et al.*, *Proc. Natl. Acad. Sci. USA,* **1994,** *91,* 8239-8243; and Santolini, et *al.*, *J. Virol.*, **1994,** *68,* 3631-3641). The mature 21 kDa core protein is cleaved from the polyprotein precursor by cellular signal peptidase and there is evidence to suggest that the HCV nucleocapsid protein is stably associated with the cytoplasmic surface of the endoplasmic reticulum membrane (Hijikata, et *al., Proc. Natl. Acad. Sci. USA,* **1991,** *88*, 5547-5551; and Santolini, *et al., J. Virol.,* **1994,** *68,* 3631-3641). In contrast to the envelope glycoproteins which include a hypervariable region in the amino-terminal region of E2 (Weiner, *et al., Virol.,* **1991,** *180,* 842-848; and Weiner, et *al., Proc. Natl. Acad. Sci. USA,* **1992,** *89,* 3468-3472), the core protein is well conserved among the different HCV genotypes and generates a host immune response (Bukh, *et al.*, *Proc. Natl. Acad. Sci. USA,* **1994,** *91,* 8239-8243; and Houghton, *et al.*, *Hepatology,* **1991, 14**, 381-388). Previous studies have shown that the majority of HCV-infected individuals develop antibodies to the HCV core protein early in the course of infection (Chiba, *et al., Proc. Natl. Acad. Sci. USA*, **1991,** *88,* 4641-4645; Hosein, *et al., Proc. Natl. Acad. Sci. USA,* **1991,** *88*, 3647-3651; Hsu, *et al.*, *Hepatology,* **1993,** *17*, 763-771; Katayama, *et al., Hepatology,* **1992,** *15,* 391-394; Nasoff, et *al., Proc. Natl. Acad. Sci. USA,* **1991,** *88,* 5462-5466; and Okamoto, *et al.*, Hepatology, **1992,** *15,* 180-186). Furthermore, the nucleocapsid protein represents an important target for the cellular immune response against HCV (Botarelli, et al., *Gastroenterol.,* **1993,** *104,* 580-587; Koziel, *et al., J. Virol.,* **1993**, *67*, 7522-7532; and Shirai, *et al.*, *J. Virol.,* **1993**, *68,* 3334-3342) . Finally, there are recent observations to suggest that the HCV core protein may have certain gene regulatory functions as well (Shih, *et al., J. Virol.,* **1993**, *67*, 5823-5832) . The high mutational rate of the viral genome probably occurs during viral replication and through immune selection. This phenomenon may be related to the establishment of persistent viral infection and subsequent disease chronicity (Weiner, *et al., Proc. Natl. Acad. Sci. USA,* **1992,** *89,* 3468-3472; Kato, *et al.*, *Biochem. Biophys. Res. Comm.,* **1992,** *189,* 119-127; and Alter., *et al., New Eng. J. Med.,* **1992,** *327,* 1899-1905).

The cellular immune events involved in liver damage and viral clearance during HCV infection have only partially been defined. In an attempt to examine a potential pathogenic role of liver-infiltrating lymphocytes in patients with chronic HCV infection, Koziel, *et al.* examined the cytotoxic T lymphocyte (CTL) response of such cells and demonstrated an HLA class I-restricted CD8+ CTL response that was directed against both structural and non-structural regions of HCV polypeptides (Koziel, *et al., J. Virol.,* **1993,** *67,* 7522-7532; and Koziel, *et al.*, *J. Immunol.,* **1992,** *149,* 3339-3344) . Other investigators have also noted the existence of CTLs in peripheral blood mononuclear cell populations that recognize epitopes on core and the other viral related proteins during chronic HCV infection (Kita, *et al.*, *Hepatol.,* **1993,** *18*, 1039-1044; and Cerny, *et al., Intl. Symp. Viral Hepatitis Liver Dis.,* **1993,** 83 (abstr.)).

Botarelli, *et al.* (Botarelli, *et al.*, *Gastroenterol.,* **1993,** *104,* 580-587) and Ferrari, *et al.* (Ferrari, *et al., Hepatol.*, **1994,** *19*, 286-295) found HLA class II-restricted CD4+ T cell-mediated proliferative responses to several recombinant proteins derived from different regions of HCV in patients with chronic HCV infection. It is noteworthy that there was a correlation between T cell responses to HCV core protein, and a clinically benign course of the liver disease as well as subsequent eradication of the virus. However, a similar study showed the proliferative response to HCV core protein did not predict a benign clinical course with respect to the severity of the liver disease (Schupper, *et al.*, *Hepatol.,* **1993,** *18,* 1055-1060). Thus, it is important to clarify the association between active cellular immunity and the clinical course of the viral infection with respect to the type of liver injury and clinical response of HCV infection to IFN therapy. In this regard, studies involving peripheral blood mononuclear cell (PBMC) responses to a recombinant GST-HCV core fusion protein were conducted, and involved evaluating the ability of such cells to produce IFN-γ; correlations were made to different clinical outcomes of HCV infection. It was found that mononuclear cells from 24 (52%) of 46 patients with chronic liver disease responded to the core protein; asymptomatic HCV carriers demonstrated a lower response rate (15%, p<0.05). More important, individuals who had received IFN-α treatment and went into clinical and virologic emission had a higher response rate (75%, p<0.05) to HCV core protein compared to those with ongoing hepatitis who failed therapy (31%). Of 25 patients whose mononuclear cells responded to HCV core protein, 18 had a significant response to one or more peptides; 12 patients reacted to a peptide mixture containing hydrophilic sequences. The core peptide amino acid sequence 140-160 was recognized by 9 patients. Interestingly, 7 of 8 patients bearing HLA DR4 and w53 haplotypes recognized the peptide sequence 141-160. Thus, the mononuclear cell response appeared to be HLA DR restricted and the responding cells were identified as CD4+ T cells. This study demonstrates the presence of immunodominant T cell epitopes within the HCV core protein in association with HLA DR phenotypes in patients with HCV associated liver disease.

Hepatitis B virus (HBV) is a major human pathogen for which there is no effective therapy. It is estimated that more than 300 million people are chronically infected with this virus worldwide. Exposure to HBV may result in acute or chronic hepatitis, liver cirrhosis and the development of hepatocellular carcinoma. The clinical consequences of this serious infection are of particular concern in the developing world where HBV infection is one of the leading causes of mortality and is also a major cause of acute and chronic liver disease in the United States and Europe as well. HBV is the prototype member of the hepadnavirus family, a group of closely related viruses (Ganem, *et al., Annu. Rev. Biochem.,* **1987,** *56,* 651-693) that including, among others, the duck hepatitis B virus (DHBV) and the woodchuck hepatitis virus (WHV) . Experimentally infected ducks or woodchucks faithfully reproduce many of the features of human disease such as acute and chronic infection and, in the case of chronically infected woodchucks, the development of hepatocellular carcinoma (Schodel, *et al.*, "The Biology of Avian Hepatitis B viruses", In Molecular Biology of the Hepatitis B Virus, 1991, Vol.3, CRC Press, Boca Raton, FL, pp.53-80; Korba, *et al.*, *J. Virol.,* **1989**, *63*, 1360-1370; and Korba, *et al.*, *Hepatology,* **1989**, *9*, 461-470). Although viral replicative intermediates have been found in other tissues, the liver is the target organ and hepatocyte injury is associated with persistent viral infection. HBV per se appears not to be a cytopathic virus. It is likely, therefore, that the host immune response produced against viral epitopes produces the liver injury and treatment strategies designed to reduce viral replication in the liver may have beneficial clinical effects.

The HBV genome encodes for 4 open reading frames (ORF) that includes: 1) the S gene encoding for the envelope protein with 2 in-frame pre-S1 and pre-S2 polypeptides; 2) the polymerase ORF encoding for a reverse transcriptase protein that is responsible for reverse transcription of a 3.6 kb pregenomic RNA into DNA; 3) the core gene encoding for a protein that is assembled to complete the viral nucleocapsid; and 4) the HBx ORF encodes for a protein of unknown function. The pol gene encompasses 80% of the genome and overlaps with the other three ORFs. The core gene is preceded by an in-frame sequence that encodes for a signal peptide and following proteolytic cleavage gives rise to an antigenically distinct protein called the HBeAg. The HBx protein was found not to be essential for the viral life cycle in vitro (Blum, et al., J. *Virol.,* **1992,** *66,* 123-127), but it appears to be necessary for the establishment of productive infection in vivo (Chen, et *al., J. Virol.,* **1993,** *67,* 1218-1226). HBx can function as a transcriptional transactivator on a variety of cellular and viral genes and suggests that it may contribute to HCC development (Schek, *et al.,* "The Hepadnaviral X Protein", *In* Molecular Biology of the Hepatitis B Virus, 1991, Vol.9, CRC Press, Boca Raton, FL, pp.181-192).

Direct injection of DNA into animals is a promising method for delivering specific antigens for immunization (Barry, *et al.*, *Bio Techniques*, **1994,** *16,* 616-619; Davis, *et al.*, *Hum. Mol. Genet.,* **1993,** *11*, 1847-1851; Tang, *et al.*, *Nature*, **1992,** *356,* 152-154; Wang, *et al., J. Virol.*, **1993,** *67,* 3338-3344; and Wolff, *et al.*, *Science*, **1990,** *247,* 1465-1468). This approach has been successfully used to generate protective immunity against influenza virus in mice and chickens, against bovine herpes virus 1 in mice and cattle and against rabies virus in mice (Cox, *et al., J. Virol.,* **1993,** *67*, 5664-5667; Fynan, *et al.*, *DNA and Cell Biol.,* **1993,** *12,* 785-789; Ulmer, *et al., Science,* **1993,** *259,* 1745-1749; and Xiang, *et al.*, *Virol.,* **1994,** *199,* 132-140). In most cases, strong, yet highly variable, antibody and cytotoxic T-cell responses were associated with control of infection. Indeed, the potential to generate long-lasting memory CTLs without using a liver vector makes this approach particularly attractive compared with those involving killed-virus vaccines and generating a CTL response that not only protects against acute infection but also may have benefits in eradicating persistent viral infection (Wolff, *et al.*, *Science,* **1990,** *247,* 1465-1468; Wolff, *et al.*, *Hum. Mol. Genet.*, **1992,** *1*, 363-369; Manthorpe, *et al.*, *Human Gene Therapy,* **1993,** *4,* 419-431; Ulmer, *et al., Science,* **1993,** *259,* 1745-1749; Yankauckas, *et al.*, *DNA and Cell Biol.,* **1993,** *12,* 777-783; Montgomery, *et al., DNA and Cell Biol.,* **1993 ,** *12,* 777-783; Fynan, *et al., DNA and Cell Biol.,* **1993,** *12*, 785-789; Wang, *et al.*, *Proc. Natl. Acad. Sci. USA,* **1993**, *90,* 4156-4160; Wang, *et al.*, *DNA and Cell Biol.,* **1993,** *12,* 799-805; Xiang, *et al., Virol.*, **1994,** *199,* 132-140; and Davis, *et al., Hum. Mol. Genet.*, **1993,** *11,* 1847-1851) of which HCV and HBV are important human diseases of world wide significance.

Presently, there is no universal, highly effective therapy of chronic HBV and/or HCV infection. Development of a vaccine strategy for HBV and/or HCV is complicated not only by the significant heterogeneity among HBV and HCV isolates, but also by the mixture of heterogeneous genomes within an isolate (Martell, *et al., J. Virol.,* **1992**, *66*, 3225). In addition, the virus contains a highly variable envelope region.

Vaccination and immunization generally refer to the introduction of a non-virulent agent against which an individual's immune system can initiate an immune response which will then be available to defend against challenge by a pathogen. The immune system identifies invading "foreign" compositions and agents primarily by identifying proteins and other large molecules which are not normally present in the individual. The foreign protein represents a target against which the immune response is made.

PCT Patent Application PCT/US90/01348 discloses sequence information of clones of the HCV genome, amino acid sequences of HCV viral proteins and methods of making and using such compositions including anti-HCV vaccines comprising HCV proteins and peptides derived therefrom.

U.S. Serial Number 08/008,342 filed January 26, 1993, U.S. Serial Number 08/029,336 filed March 11, 1993, U.S. Serial Number 08/125,012 filed September 21, 1993, PCT Patent Application Serial Number PCT/US94/00899 filed January 26, 1994, and U.S. Serial Number 08/221,579 filed April 1, 1994 each contains descriptions of genetic immunization protocols. Vaccines against HCV are disclosed in each.

U.S. Serial Number 08/318,248 filed October 5, 1994, discloses genetic constructs comprising nucleotide sequences encoding HCV core protein which are useful as a vaccine. The HCV core DNA-based vaccine expresses high levels of core antigen in vitro and induces a strong immune response in vivo.

There remains a need for vaccines useful to protect individuals against hepatitis B virus and/or hepatitis C virus infection. There remains a need for pharmaceutical compositions for protecting individuals against hepatitis B virus and/or hepatitis C virus infection.

### SUMMARY OF THE INVENTION

According to a first aspect of the present invention there is provided a recombinant nucleic acid molecule comprising a nucleotide coding sequence that encodes a fusion protein, said fusion protein comprising hepatitis B virus S gene product linked to amino acids 1-69 of hepatitis C virus core protein.

Preferably, said fusion protein comprises hepatitis B virus S gene product linked to amino acids 1-70 to 1-154 of hepatitis C virus core protein.

Conveniently, said fusion protein comprises a fragment of hepatitis B virus pre S2 gene product linked to hepatitis B virus S gene product linked to amino acids 1-69 of hepatitis C virus core protein.

In a preferred embodiment, said fusion protein comprises a fragment of hepatitis B virus pre S2 gene product linked to hepatitis B virus S gene product linked amino acids 1-70 to 1-154 of hepatitis C virus core protein.

Preferably, said fusion protein comprises a fragment of hepatitis B virus pre S1 gene product linked to a fragment of hepatitis B virus pre S2 gene product linked to hepatitis B virus S gene product linked to amino acids 1-69 of hepatitis C virus core protein.

In a preferred embodiment, said fusion protein comprises a fragment of hepatitis B virus pre S1 gene product linked to a fragment of hepatitis B virus pre S2 gene product linked to hepatitis B virus S gene product linked to amino acids 1-70 to 1-154 of hepatitis C virus core protein.

Preferably, said nucleotide coding sequence is operably linked to cytomegalovirus promoter, Rous Sarcoma Virus enhancer, polyadenylation sequence, and optionally 5' UTR of hepatitis C virus.

According to a second aspect of the present invention there is provided a pharmaceutical composition comprising:
a) a recombinant nucleic acid molecule comprising a nucleotide coding sequence that encodes a fusion protein, said fusion protein comprising a hepatitis B virus S gene product linked to amino acids 1-69 of hepatitis C virus core protein, wherein said nucleotide coding sequence is operably linked to regulatory elements functional in human cells; and
b) a pharmaceutically acceptable carrier or diluent.

Conveniently, said fusion protein comprises hepatitis B virus S gene product linked to amino acids 1-70 to 1-154 of hepatitis C virus core protein.

Preferably, said fusion protein comprises a fragment of hepatitis B virus pre S2 gene product linked to hepatitis B virus S gene product linked to amino acids 1-69 of hepatitis C virus core protein.

In a preferred embodiment, said fusion protein comprises a fragment of hepatitis B virus pre S2 gene product linked to hepatitis B virus S gene product linked to amino acids 1-70 to 1-154 of hepatitis C virus core protein.

Conveniently, said fusion protein comprises a fragment of hepatitis B virus pre S1 gene product linked to a fragment of hepatitis B virus pre S2 gene product linked to hepatitis B virus S gene product linked to amino acids 1-69 of hepatitis C virus core protein.

Preferably, said fusion protein comprises a fragment of hepatitis B virus pre S1 gene product linked to a fragment of hepatitis B virus pre S2 gene product linked to hepatitis B virus S gene product linked to amino acids 1-70 to 1-154 of hepatitis C virus core protein.

In a preferred embodiment said regulatory elements functional in human cells comprise cytomegalovirus promoter, Rous Sarcoma Virus enhancer, - polyadenylation sequence, and optionally 5' UTR of hepatitis C virus.

According to a third aspect of the present invention there is provided an isolated and purified nucleic acid molecule having a nucleotide sequence comprising an incomplete hepatitis C viral genome, said genome comprising a coding sequence for a hepatitis C virus core protein and a 5' untranslated region wherein said 5' untranslated region is free of the 5' untranslated region of hepatitis C virus except the last 9 nucleotides of said 5' untranslated region.

Conveniently, said nucleic acid molecule is plasmid pHCV2-1.

According to a fourth aspect of the present invention there is provided an isolated and purified nucleic acid molecule consisting of a coding sequence encoding the hepatitis C virus core protein and one or more regulatory elements operably linked to said nucleotide sequence encoding the hepatitis C virus core protein.

Conveniently, said coding sequence comprises SEQ ID NO:15.

According to a fifth aspect of the present invention there is provided an isolated and purified nucleic acid molecule having a nucleotide sequence encoding SEQ ID NO:15.

Preferably, said nucleotide sequence comprises at least the last 9 nucleotides of the 5' untranslated region of hepatitis C virus.

In a preferred embodiment, said nucleotide sequence comprises the entire 5' untranslated region of hepatitis C virus.

Conveniently, the nucleic acid molecule further comprises a promoter and polyadenylation sequence and said SEQ ID NO:15 is operably linked to said promoter and polyadenylation sequence.

Preferably, the nucleic acid molecule further comprises a promoter and polyadenylation sequence and said coding sequence is operably linked to said promoter and polyadenylation sequence.

Conveniently, the nucleic acid molecule further comprises an enhancer, wherein said coding sequence is operably linked to said enhancer.

Preferably, the nucleic acid molecule further comprises a Kozak sequence, wherein said coding sequence is operably linked to said Kozak sequence.

According to a sixth aspect of the present invention there is provided a pharmaceutical composition comprising a nucleic acid module of the present invention and a pharmaceutically acceptable carrier or diluent.

### BRIEF DESCRIPTION OF THE FIGURES

Figure 1 shows a schematic of the plurality of constructs comprising the HCV core gene, the HBV S gene, the HBV pre S1 gene, and the HBV pre S2 gene.
Figure 2 shows a diagram of an expression vector into which coding sequences enclosing a chimeric HCV/HBV fusion protein can be inserted, or into which HCV coding sequence is inserted to produce plasmids pHCV2-1 and pHCV4-2.
Figure 3 depicts methods for constructing the HBV/HCV fusion constructs.

### DETAILED DESCRIPTION OF THE INVENTION

According to the present invention, compositions are provided which prophylactically and/or therapeutically immunize an individual against HBV and/or HCV infection. For immunization against HBV and/or HCV infection, recombinant nucleic acid molecules comprising a nucleotide coding sequence that encodes a fusion protein that comprises HBV S gene product linked to amino acids 1-69 of HCV core protein can be administered to the individual. The fusion protein encoded by the gene construct is expressed by the individual's cells and serves as an immunogenic target against which an anti-HBV and/or anti-HCV immune responses are elicited. For immunization against HCV infection, genetic material constituting an incomplete hepatitis C viral genome but which encodes HCV core protein can be administered to the individual. The protein encoded by the gene construct is expressed by the individual's cells and serves as an immunogenic target against which an anti-HCV immune response is elicited. The resulting immune responses are broad based; in addition to a humoral immune response, both arms of the cellular immune response are elicited. The present invention is useful for conferring prophylactic and therapeutic immunity. Thus, a immunizing includes both protecting an individual from HBV and/or HCV challenge as well as treating an individual suffering from HBV and/or HCV infection.

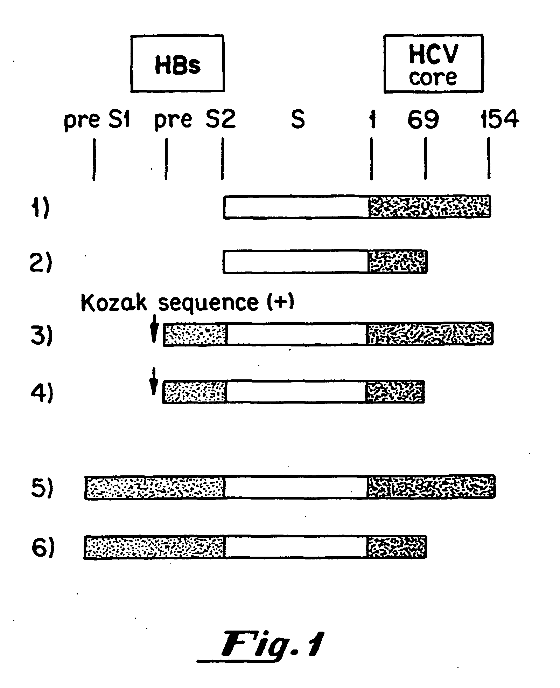

It has been shown that many proteins previously known to induce a humoral and cellular immune responses following DNA immunization have either been native cell surface proteins or secreted proteins, such as, for example, influenza NP, HBsAg, and rabies virus glycoproteins. Although HCV core DNA-based vaccine expresses high levels of core antigen *in vitro* and induces an immune response *in vivo,* it is likely that the HCV core protein remains anchored in the cytoplasm and is not expressed on the surface of the cell or secreted from the cell. Therefore, the immune response may be increased by providing for cell surface expression and/or secretion of the protein. Accordingly, in one embodiment of the invention, the nucleotide coding sequence encoding HCV core protein was linked to regions of the nucleotide coding sequence encoding HBV surface antigen, as shown in Figure 1. Thus, the chimeric HBV/HCV fusion protein can elicit an immune response to HBV and/or HCV.

As used herein, the term "incomplete hepatitis C viral genome" is meant to refer to a nucleic acid molecule that does not contain the complete coding sequence for the HCV polyprotein which is encoded by the HCV viral genome. Incorporation of an incomplete HCV genome into a cell does not constitute introduction of sufficient genetic information for the production of infectious virus.

As used herein the term "HCV core protein" is meant to refer to truncated HCV core proteins, such as those having the amino acid sequence set forth in SEQ ID NO:1 and SEQ ID NO:2, which comprises 154 amino acids, or the full-length HCV core protein having the amino acid sequence disclosed in SEQ ID NO:14 and SEQ ID NO:15 which is the amino acid sequence of the HCV core protein of a specific HCV isolate. The full-length HCV core protein normally has 191 amino acids, but for the purposes of the present invention relating to the truncated HCV core protein, the C-terminal 37 amino acids (amino acids 155-191) have been deleted to enhance the ability of the fusion protein to be secreted. The C-terminal twenty amino acids of the full-length HCV core protein are postulated to contain the binding sites to the endoplasmic reticulum, resulting in the retention of the core protein inside the cell cytoplasm. In addition, the term HCV core protein is meant to refer to corresponding HCV core proteins, full-length or truncated, from additional HCV isolates which may vary. Those having ordinary skill in the art can readily identify the HCV core protein from additional HCV isolates. Nucleotide and amino acid sequences for HCV core protein have been disclosed in U.S. Serial Number 08/318,248 filed October 5, 1994. It is to be understood that nucleotide substitutions in the codon may be acceptable when the same amino acid is encoded. In addition, it is also to be understood that nucleotide changes may be acceptable wherein a conservative amino acid substitution results from the nucleotide substitution.

The HCV core protein has been observed to exist as two distinct forms, the anchored core protein and the virion core protein. During HCV translation, a precursor polypeptide is translated and subsequently processed by cellular proteases and viral proteases to yield the individual viral polypeptides. A cellular signal peptidase is believed to cleave at a site which separates the portion of the polyprotein that becomes the core protein from the portion that becomes the envelope protein, thereby releasing anchored core protein from the envelope protein. Anchored core protein, which is associated with the endoplasmic reticulum, is further processed to mature virion core. Anchored core protein differs from virion core in that it contains an extra 18 amino acids at the C terminus. Virtually no anchored core is found on viral particles.

As used herein, the term "gene construct" is meant to refer to a recombinant nucleic acid molecule comprising a nucleotide coding sequence that encodes a fusion protein that comprises at least the N-terminal 69 amino acids of the HCV core protein and the entire HBV S gene product, or the full-length HCV core protein, as well as initiation and termination signals operably linked to regulatory elements including a promoter and polyadenylation signal capable of directing expression in the cells of the vaccinated individual. In some embodiments, the gene construct further comprises an enhancer, Kozak sequence (GCCGCCATG SEQ ID NO:13) , and at least a fragment of the HCV 5' UTR.

As used herein, the term "genetic vaccine" refers to a pharmaceutical preparation that comprises a gene construct. Genetic vaccines include pharmaceutical preparations useful to invoke a prophylactic and/or therapeutic immune responseto HCV and/or HBV.

According to the present invention, gene constructs are introduced into the cells of an individual where it is expressed, thus producing a HCV core/HBV surface antigen fusion protein or a full-length HCV core protein. The regulatory elements of the gene constructs of the invention are capable of directing expression in human cells. The regulatory elements include a promoter and a polyadenylation signal. In addition, other elements, such as an enhancer and a Kozak sequence, may also be included in the gene construct.

When taken up by a cell, the gene constructs of the invention may remain present in the cell as a functioning extrachromosomal molecule or it may integrate into the cell's chromosomal DNA. DNA may be introduced into cells where it remains as separate genetic material in the form of a plasmid. Alternatively, linear DNA which can integrate into the chromosome may be introduced into the cell. When introducing DNA into the cell, reagents which promote DNA integration into chromosomes may be added. DNA sequences which are useful to promote integration may also be included in the DNA molecule. Alternatively, RNA may be administered to the cell. It is also contemplated to provide the gene construct as a linear minichromosome including a centromere, telomeres and an origin of replication.

In one embodiment of the present invention, the gene construct comprises recombinant nucleic acid molecules comprising a nucleotide coding sequence that encodes a fusion protein that comprises regions of the HBV S gene product linked to HCV core protein, preferably truncated HCV core protein.

In some preferred embodiments, the recombinant nucleic acid molecule comprises a nucleotide coding sequence that encodes a fusion protein that comprises the entire HBV S gene product linked to amino acids 1-69 of HCV core protein. The nucleotides encoding the C-terminal amino acids of HBV S gene product are linked to the nucleotides encoding the N-terminal amino acids of truncated HCV core protein.

In some preferred embodiments, the recombinant nucleic acid molecule comprises a nucleotide coding sequence that encodes a fusion protein that comprises the entire HBV S gene product linked to amino acids 1-70 to 1-154 of truncated HCV core protein. The truncated HCV core protein may comprise amino acids 1-70, 1-71, 1-72, 1-73, 1-74...1-150, 1-151, 1-152, 1-153, or 1-154. As used herein "1-70 to 1-154" and "1-70, 1-71, 1-72, 1-73, 1-74 ... 1-150, 1-151, 1-152, 1-153, or 1-154" are used interchangeably and are meant to describe each of the 83 amino acids sequences of fragments of truncated HCV core protein that include 1-69 plus each additional C terminal residue up to 154. The nucleotides encoding the C-terminal amino acids of HBV S gene product are linked to the nucleotides encoding the N-terminal amino acids of truncated HCV core protein.

In some preferred embodiments, the recombinant nucleic acid molecule comprises a nucleotide coding sequence that encodes a fusion protein that comprises a fragment of HBV pre S2 gene product linked to the entire HBV S gene product linked to amino acids 1-69 of truncated HCV core protein. A fragment of HBV pre S2 gene product may include the C-terminal amino acid, or the C-terminal 2 amino acids, or the C-terminal 3 amino acids, or the C-terminal 4 amino acids...or the entire HBV pre S2 gene product. As used herein, "a fragment of HBV pre S2 gene product may include the C-terminal amino acid, or the C-terminal 2 amino acids, or the C-terminal 3 amino acids, or the C-terminal 4 amino acids...or the entire HBV pre S2 gene product" is meant to describe amino acid sequences that include fragments of the HBV pre S2 starting with the fragment that includes only the C terminal residue to fragments that include additional residues immediately N terminal to the C terminal residue, i.e. the last C terminal residue, the last two C terminal residues, the last three C terminal residues and so on until the entire pre S2 protein is present. The nucleotides encoding the C-terminal amino acids of the HBV pre S2 gene product are linked to the nucleotides encoding the N-terminal amino acids of the HBV S gene product. The nucleotides encoding the C-terminal amino acids of the HBV S gene product are in turn linked to the nucleotides encoding the N-terminal amino acids of truncated HCV core protein.

In some preferred embodiments, the recombinant nucleic acid molecule comprises a nucleotide coding sequence that encodes a fusion protein that comprises a fragment of HBV pre S2 gene product linked to the entire HBV S gene product linked to amino acids 1-70 to 1-154 of truncated HCV core protein. A fragment of HBV pre S2 gene product may include the C-terminal amino acid, or the C-terminal 2 amino acids, or the C-terminal 3 amino acids, or the C-terminal 4 amino acids...or the entire HBV pre S2 gene product. The truncated HCV core protein may comprise amino acids 1-70, 1-71, 1-72, 1-73, 1-74...1-150, 1-151, 1-152, 1-153, or 1-154. The nucleotides encoding the C-terminal amino acids of the HBV pre S2 gene product are linked to the nucleotides encoding the N-terminal amino acids of the HBV S gene product. The nucleotides encoding the C-terminal amino acids of the HBV S gene product are in turn linked to the nucleotides encoding the N-terminal amino acids of truncated HCV core protein.

In some preferred embodiments, the recombinant nucleic acid molecule comprises a nucleotide coding sequence that encodes a fusion protein that comprises a fragment of HBV pre S1 gene product linked to a fragment of HBV pre S2 gene product linked to the entire HBV S gene product linked to amino acids 1-69 of truncated HCV core protein. A fragment of HBV pre S1 gene product may include the C-terminal amino acid, or the C-terminal 2 amino acids, or the C-terminal 3 amino acids, or the C-terminal 4 amino acids...or the entire HBV pre S1 gene product. As used herein, "a fragment of HBV pre S1 gene product may include the C-terminal amino acid, or the C-terminal 2 amino acids, or the C-terminal 3 amino acids, or the C-terminal 4 amino acids...or the entire HBV pre S1 gene product" is meant to describe amino acid sequences that include fragments of the HBV pre S1 starting with the fragment that includes only the C terminal residue to fragments that include additional residues immediately N terminal to the C terminal residue, *i.e.* the last C terminal residue, the last two C terminal residues, the last three C terminal residues and so on until the entire pre S1 protein is present. A fragment of HBV pre S2 gene product may include the C-terminal amino acid, or the C-terminal 2 amino acids, or the C-terminal 3 amino acids, or the C-terminal 4 amino acids...or the entire HBV pre S2 gene product. The nucleotides encoding the C-terminal amino acids of HBV pre S1 gene product are linked to the nucleotides encoding the N-terminal amino acids of HBV pre S2 gene product . The nucleotides encoding the C-terminal amino acids of the HBV pre S2 gene product are in turn linked to the nucleotides encoding the N-terminal amino acids of HBV S gene product. The nucleotides encoding the C-terminal amino acids of HBV S gene product are in turn linked to the nucleotides encoding the N-terminal amino acids of truncated HCV core protein.

In some preferred embodiments, the recombinant nucleic acid molecule comprises a nucleotide coding sequence that encodes a fusion protein that comprises a fragment of HBV pre S1 gene product linked to a fragment of HBV pre S2 gene product linked to HBV S gene product linked to amino acids 1-70 to 1-154 of truncated HCV core protein. The truncated HCV core protein may comprise amino acids 1-70, 1-71, 1-72, 1-73, 1-74...1-150, 1-151, 1-152, 1-153, or 1-154. A fragment of HBV pre S1 gene product may include the C-terminal amino acid, or the C-terminal 2 amino acids, or the C-terminal 3 amino acids, or the C-terminal 4 amino acids...or the entire HBV pre S1 gene product. A fragment of HBV pre S2 gene product may include the C-terminal amino acid, or the C-terminal 2 amino acids, or the C-terminal 3 amino acids, or the C-terminal 4 amino acids...or the entire HBV pre S2 gene product. The nucleotides encoding the C-terminal amino acids of HBV pre S1 gene product are linked to the nucleotides encoding the N-terminal amino acids of HBV pre S2 gene product. The nucleotides encoding the C-terminal amino acids of HBV pre S2 gene product are in turn linked to the nucleotides encoding the N-terminal amino acids of HBV S gene product. The nucleotides encoding the C-terminal amino acids of HBV S gene product are in turn linked to the nucleotides encoding the N-terminal amino acids of truncated HCV core protein.

It is contemplated that the nucleotide coding sequences may be linked to one another with or without nucleotide spacers or linkers, such that the downstream nucleotide coding sequence remains in-frame. Thus, the nucleotide coding sequences may be linked directly, that is, without any nucleotide spacers. Alternatively, a spacer comprising from about 3 to about 60 nucleotides may be inserted between adjacent nucleotide coding sequences. For example, a nucleotide spacer may be inserted between the nucleotide coding sequences for HBV S gene and truncated HCV core gene, between HBV pre S2 gene HBV S gene, and HBV pre S1 gene and HBV pre S2 gene. The spacer or linker may comprise restriction endonuclease sites for cloning purposes.

It is also contemplated that the recombinant nucleic acid molecule comprising a nucleotide coding sequence that encodes a fusion protein that may comprise less than the entire HBV S gene product linked to amino acids 1-69 of truncated HCV core protein, without substantially altering the effectiveness of the vaccine. It is also contemplated that nucleotide substitutions may be made throughout the nucleotide coding sequence without affecting the amino acid sequence of the fusion protein. It is also contemplated that conservative amino acid substitutions may be made throughout the fusion protein without substantially reducing the immunogenic activity of the fusion protein.

In some preferred embodiments, the gene construct includes at least a fragment of the HCV 5' UTR including the last 9 nucleotides of the HCV 5' UTR. As used herein, the term "the last 9 nucleotides of the HCV 5' UTR" is meant to refer to the 9 most 3' nucleotides of the 5' UTR. That is, the 9 nucleotides of the 5' UTR that immediately precede the coding sequence. The last 9 nucleotides of the HCV 5' UTR of a preferred embodiment is shown as SEQ ID NO:3.

In some embodiments, the fragment of the 5' UTR that includes the last 9 nucleotides of the HCV 5' UTR comprises the last 25 nucleotides of the HCV 5' UTR. In some embodiments, the fragment of the 5' UTR that includes the last 9 nucleotides of the HCV 5' UTR comprises the last 50 nucleotides of the HCV 5' UTR. In some embodiments, the fragment of the 5' UTR that includes the last 9 nucleotides of the HCV 5' UTR comprises the last 75 nucleotides of the HCV 5' UTR. In some embodiments, the fragment of the 5' UTR that includes the last 9 nucleotides of the HCV 5' UTR comprises the last 100 nucleotides of the HCV 5' UTR. In some embodiments, the fragment of the 5' UTR that includes the last 9 nucleotides of the HCV 5' UTR comprises the last 150 nucleotides of the HCV 5' UTR. In some embodiments, the fragment of the 5' UTR that includes the last 9 nucleotides of the HCV 5' UTR comprises the last 200 nucleotides of the HCV 5' UTR. In some embodiments, the fragment of the 5' UTR that includes the last 9 nucleotides of the HCV 5' UTR comprises the last 250 nucleotides of the HCV 5' UTR. In some embodiments, the fragment of the 5' UTR that includes the last 9 nucleotides of the HCV 5' UTR comprises the last 300 nucleotides of the HCV 5' UTR.

In some preferred embodiments, the gene construct includes the entire HCV 5' UTR. In some preferred embodiments, the gene construct includes the 9 most 3' nucleotides of the HCV 5' UTR. The entire HCV 5' UTR of a preferred embodiment is shown as SEQ ID NO:4.

According to other aspects of the invention, the gene construct includes coding sequences which encode the full-length HCV core protein. In some preferred embodiments, the gene construct encodes an anchored form of the full-length HCV core protein. Anchored core protein is preferred in some embodiments for the following reasons. First, it is believed to be desirable to correctly compartmentalize the core protein to the endoplasmic reticulum where the core protein is first found in naturally infected cells. If a protein is incorrectly compartmentalized, there is a risk of lowering total expression of that protein due to degradation or poisoning of the cell. Further, since a cellular protease is thought to cleave anchored core to virion core, both species may be present in cells expressing the anchored form. If there are differences in the antigenicity between the two species, a broader immune response against core is potentially generated by expressing the anchored form of full-length core protein. Moreover, the extra 18 amino acids in the anchored core may contain either antibody epitopes or cytotoxic T cell epitopes or both. Expression of the anchored form provides the presence of these regions which may broaden the immune response to core protein.

In some preferred embodiments, the gene construct encodes a full-length HCV core protein which consists of the amino acid sequence of SEQ ID NO:14 and SEQ ID NO:15. In some preferred embodiments, the gene construct comprises the coding sequence in SEQ ID NO:14.

In some preferred embodiments, the gene construct comprises SEQ ID NO:14.

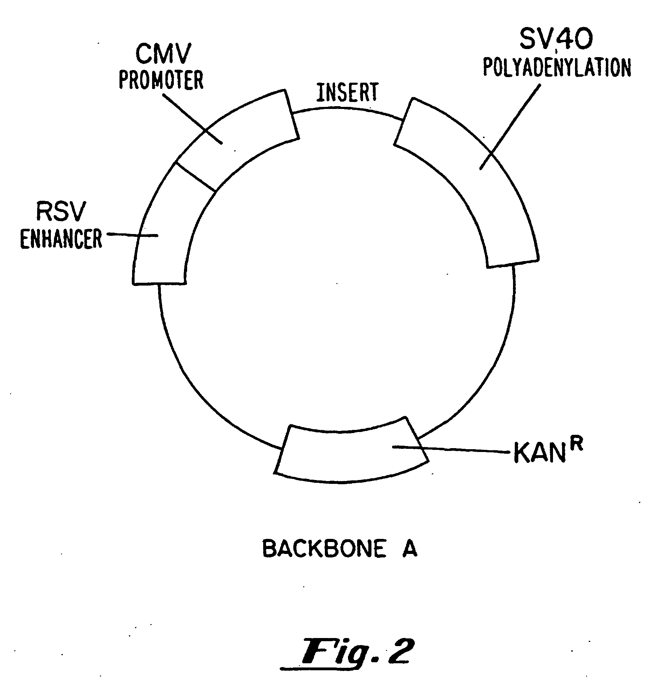

In some preferred embodiments, the vector used is selected from those described in Figure 2. In some embodiments, nucleotides 342 to 923 of SEQ ID NO:14 is inserted into backbone A to form plasmid pHCV2-1. In some embodiments, SEQ ID NO:14 is inserted into backbone A to form plasmid pHCV4-2.

In plasmids pHCV2-1 and pHCV4-2, coding sequence encoding the full-length HCV core protein is under the regulatory control of the CMV immediate early promoter and the SV40 minor polyadenylation signal. Constructs may optionally contain the SV40 origin of replication.

The regulatory elements necessary for gene expression of a DNA molecule include: a promoter, an initiation codon, a stop codon, and a polyadenylation signal. In addition, enhancers are often required for gene expression. It is necessary that these elements be operably linked to the sequence that encodes the fusion protein or the full-length HCV core protein and that the regulatory elements are operable in the individual to whom they are administered.

Initiation codons and stop codon are generally considered to be part of a nucleotide sequence that encodes the fusion protein or the full-length HCV core protein.

Promoters and polyadenylation signals used must be functional within the cells of the individual. In order to maximize protein production, regulatory sequences may be selected which are well suited for gene expression in the cells the construct is administered into. Moreover, codons may be selected which are most efficiently transcribed in the cell. One having ordinary skill in the art can produce DNA constructs which are functional in the cells.

Examples of promoters useful to practice the present invention, especially in the production of a genetic vaccine for humans, include but are not limited to promoters from Simian Virus 40 (SV40) , Mouse Mammary Tumor Virus (MMTV) promoter, Human Immunodeficiency Virus (HIV) such as the HIV Long Terminal Repeat (LTR) promoter, Moloney virus, ALV, Cytomegalovirus (CMV) such as the CMV immediate early promoter, Epstein Barr Virus (EBV), Rous Sarcoma Virus (RSV) as well as promoters from human genes such as human Actin, human Myosin, human Hemoglobin, human muscle creatine and human metalothionein.

Examples of polyadenylation signals useful to practice the present invention, especially in the production of a genetic vaccine for humans, include but are not limited to SV40 polyadenylation signals and LTR polyadenylation signals. In particular, the SV40 polyadenylation signal which is in pCEP4 plasmid (Invitrogen, San Diego CA) , referred to as the SV40 polyadenylation signal, is used.

In addition to the regulatory elements required for gene expression, other elements may also be included in a gene construct. Such additional elements include enhancers. The enhancer may be selected from the group including but not limited to: human Actin, human Myosin, human Hemoglobin, human muscle creatine and viral enhancers such as those from CMV, RSV and EBV.

Gene constructs can be provided with mammalian origin of replication in order to maintain the construct extrachromosomally and produce multiple copies of the construct in the cell. Plasmids pCEP4 and pREP4 from Invitrogen (*San* Diego, CA) contain the Epstein Barr virus origin of replication and nuclear antigen EBNA-1 coding region which produces high copy episomal replication without integration.

In some preferred embodiments, the gene construct used is selected from the vectors described in Figure 2. In some embodiments, nucleotide coding sequence encoding the fusion protein is inserted into backbone A.

In expression vectors of the invention, nucleotide coding sequence encoding the fusion protein is under the regulatory control of the CMV immediate early promoter and the SV40 minor polyadenylation signal. Constructs may optionally contain the SV40 origin of replication.

Routes of administration include, but are not limited to, intramuscular, intraperitoneal, intradermal, subcutaneous, intravenous, intraarterially, intraoccularly and oral as well as transdermally or by inhalation or suppository. Preferred routes of administration include intramuscular, intraperitoneal, intradermal and subcutaneous injection. Delivery of gene constructs which encode chimeric HBV/HCV protein or full-length HCV core protein can confer mucosal immunity in individuals immunized by a mode of administration in which the material is presented in tissues associated with mucosal immunity. Thus, in some examples, the gene construct is delivered by administration in the buccal cavity within the mouth of an individual.

Gene constructs may be administered by means including, but not limited to, traditional syringes, needleless injection devices, or "microprojectile bombardment gene guns". Alternatively, the genetic vaccine may be introduced by various means into cells that are removed from the individual. Such means include, for example, *ex vivo* transfection, electroporation, microinjection and microprojectile bombardment. After the gene construct is taken up by the cells, they are reimplanted into the individual. It is contemplated that otherwise non-immunogenic cells that have gene constructs incorporated therein can be implanted into the individual even if the vaccinated cells were originally taken from another individual.

The gene construct of the present invention can be administered to an individual using a needleless injection device. The gene construct of the present invention can be simultaneously administered to an individual intradermally, subcutaneously and intramuscularly using a needleless injection device. Needleless injection devices are well known and widely available. One having ordinary skill in the art can, following the teachings herein, use needleless injection devices to deliver genetic material to cells of an individual. Needleless injection devices are well suited to deliver genetic material to all tissue. They are particularly useful to deliver genetic material to skin and muscle cells. In some embodiments, a needleless injection device may be used to propel a liquid that contains DNA molecules toward the surface of the individual's skin. The liquid is propelled at a sufficient velocity such that upon impact with the skin the liquid penetrates the surface of the skin, permeates the skin and muscle tissue therebeneath. Thus, the genetic material can be simultaneously administered intradermally, subcutaneously and intramuscularly. In some embodiments, a needleless injection device may be used to deliver genetic material to tissue of other organs in order to introduce a nucleic acid molecule to cells of that organ. Genetic vaccines according to the present invention comprise about 1 nanogram to about 1000 micrograms of DNA. In some preferred embodiments, the vaccines contain about 10 nanograms to about 800 micrograms of DNA. In some preferred embodiments, the vaccines contain about 0.1 to about 500 micrograms of DNA. In some preferred embodiments, the vaccines contain about 1 to about 350 micrograms of DNA. In some preferred embodiments, the vaccines contain about 25 to about 250 micrograms of DNA. In some preferred embodiments, the vaccines contain about 100 micrograms DNA.

The genetic vaccines according to the present invention are formulated according to the mode of administration to be used. One having ordinary skill in the art can readily formulate a pharmaceutical composition that comprises a gene construct. In some cases, an isotonic formulation is used. Generally, additives for isotonicity can include sodium chloride, dextrose, mannitol, sorbitol and lactose. In some cases, isotonic solutions such as phosphate buffered saline are preferred. Stabilizers include gelatin and albumin. In some embodiments, a vasoconstriction agent is added to the formulation. The pharmaceutical preparations according to the present invention are provided sterile and pyrogen free.

The gene constructs of the invention may be formulated with or administered in conjunction with agents that increase uptake and/or expression of the gene construct by the cells relative to uptake and/or expression of the gene construct by the cells that occurs when the identical genetic vaccine is administered in the absence of such agents. Such agents and the protocols for administering them in conjunction with gene constructs are described in U.S. Serial Number 08/008,342 filed January 26, 1993, U.S. Serial Number 08/029,336 filed March 11, 1993, U.S. Serial Number 08/125,012 filed September 21, 1993, PCT Patent Application Serial Number PCT/US94/00899 filed January 26, 1994, and U.S. Serial Number 08/221,579 filed April 1, 1994. Examples of such agents include: CaPO₄, DEAE dextran, anionic lipids; extracellular matrix-active enzymes; saponins; lectins; estrogenic compounds and steroidal hormones; hydroxylated lower alkyls; dimethyl sulfoxide (DMSO); urea; and benzoic acid esters anilides, amidines, urethanes and the hydrochloride salts thereof such as those of the family of local anesthetics. In addition, the gene constructs are encapsulated within/administered in conjunction with lipids/polycationic complexes.

### EXAMPLES

### Example 1: Design and construction of HBV/HCV expression plasmids

A large S HB clone containing the entire pre S1, pre S2, and *S* sequences *(adr-*1*)* was established. The nucleotide and amino acid sequences of the pre S1/pre S2/S gene is depicted in SEQ ID NO:5. Briefly, 100 µl of serum obtained from a patient with HBV related chronic hepatitis was incubated at 70°C for 3 hours in a mixture of proteinase K (100 µg/ml) , 0.5% SDS, 5 mM EDTA, and 10 mm Tris-HCl, pH 8.0. The solution was extracted with phenol-chloroform and the DNA precipitated with ethanol. A PCR reaction was conducted in a 100 µl mixture containing 10 µl of serum DNA sample, 2.5 U Taq polymerase (Perkin-Elmer Cetus, Norwalk, CT) , 50 µM of each dNTP, and 0.4 µM of primer HBs-3 5' -GGGTCACCATATTCTTGGGAA-3' (SEQ ID NO:6) and HBs-1 5'-GCAGCAAAGCCCAAAAGACCC-3' (SEQ ID NO:7) . The reaction mixture was cycled 35 times. Amplified DNA was cloned into pCR™ vector using TA cloning kit (Invitrogen). The HCV clone (TH, genotype II) was also established from a patient with chronic hepatitis C infection as described by Wakita, *et al., J. Biol. Chem.,* **1994,** *269,* 1405-1410.

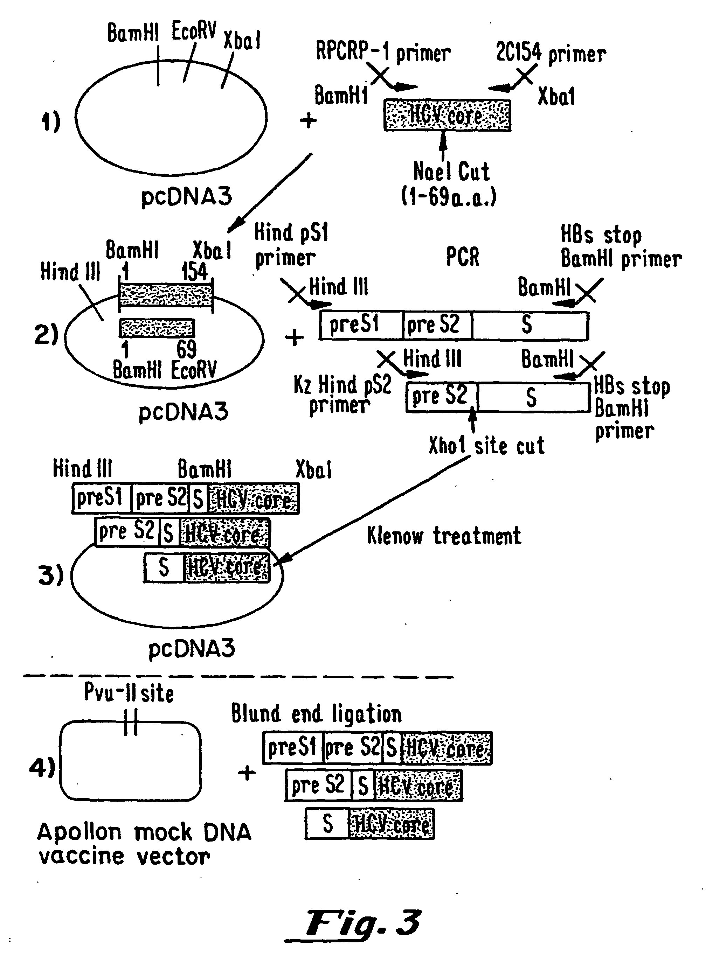

Figure 3 depicts preferred methods used to prepare the HCV/HBV fusion constructs. The first step required PCR amplification of the truncated HCV core gene. In brief, 10 µg of TH-DNA was amplified by RPCRP-1 primer 5'-CGGGATCCATGAGCACGAATCCTAAACC-3' (SEQ ID NO:8) and 2C154XBA-R 5 ' -TCTCTAGATTACTAGCCATGCGCCAAGGCCCTGG- 3' (SEQ ID NO:9) primer. The PCR product was digested with BamHI and XbaI, and ligated into the pcDNA3 vector (Invitrogen). The final 20 amino acids of the truncated HCV core protein binds tightly to ER membrane. Considering this information, with respect to fusion protein secretion, the final 37 amino acids of the full-length HCV core protein (amino acids 155 to 191) were deleted. Another plasmid encoding a shortened or truncated HCV core gene (HCV core amino acids 1 to 69) was also produced because it was possible that the fusion proteins containing truncated HCV core amino acids 1 to 154 will be too long to be secreted. To make the shortened or truncated HCV construct, amplified DNA was digested with BamHI and NaeI and ligated into BamHI site-EcoRl site in the pcDNA3 vector.

Finally, the PCR of the HBs product was produced between the HBs stop BamHI primer 5'-CATGGATCCAATGTATACCCAAAGACA-3' (SEQ ID NO:10) and Hind pS1 primer 5' -AGACACAAGCTTATGGGAGGTTGGTCTTCCAAAC-3' (SEQ ID NO:11) or Kz Hind pS2 primer 5'-AGACACAAGCTTGCCGCCATGCAGTGGAACT-3' (SEQ ID NO:12) . Amplified DNA was digested with BamHI and HindIII, and ligated into the BamHI site-HindIII site of the pcDNA3 vector that included the HCV core gene. The gene encoding the small S was produced by digestion of middle S (pre S2-S) PCR product by Xho-I followed by Klenow treatment. In the upstream sequence of the pre-S2-S-HCV fusion constructs, a Kozak sequence (GCCGCCATG SEQ ID NO:13) was included in the Kz Hind pS2 primer and this was added to the preS2-S-HCV construct since the pre-S2-S-HCV construct without the Kozac sequence was frequently translated from the ATG codon of HBs gene (small S) and not from the ATG codon of pre S2 gene. As shown in Figure 1, six fusion constructs were prepared. Complete sequence analysis of all constructs was performed and no PCR-induced mutations were found. Finally, these six HB-HCV inserts were ligated to Pvu-II site of vaccine expression vector.

One skilled in the art having the DNA sequences encoding the truncated HCV core protein, the pre S1 protein, the pre S2 protein, and the S protein can design primers for preparing any of the chimeric gene constructs of the present invention. In addition, nucleotide base substitutions may be made without affecting the binding of the primers. Moreover, the primers may be prepared with endonuclease restriction sites for cloning and ligating purposes, as known to those skilled in the art. Thus, one skilled in the art can prepare any of the gene constructs of the present invention by designing the appropriate primers and performing PCR amplification. The PCR products are ligated into an expression vector.

Plasmids comprising the nucleotide coding sequence for the HBV/HCV fusion proteins described above each contain the nucleotide coding region for the fusion protein placed under the transcriptional control of the CMV promoter and the RSV enhancer element.

Plasmid backbone A is 3969 base pairs in length. It contains a PBR origin of replication for replicating in E. coli. It also contains a kanamycin resistance gene so that the plasmid can be selected in E. coli. Inserts such as the truncated or full-length HCV core gene, are cloned into a polylinker region which places the insert between and operably linked to the promoter and polyadenylation signal. Transcription of the cloned inserts is under the control of the CMV promoter and the RSV enhancer elements. A polyadenylation signal is provided by the presence of an SV40 poly A signal situated just 3' of the cloning site.

### Example 2: Design and construction of full-length HCV expression plasmids

Plasmids, pHCV2-1 and pHCV4-2, were both designed to express the anchored core protein of HCV. Anchored core, the cellular precursor to virion core, remains membrane associated by means of a hydrophobic carboxy terminus.

Each plasmid construct contains the full-length HCV core coding region placed under the transcriptional control of the CMV promoter and the RSV enhancer element. Plasmid pHCV2-1 contains a 9 nucleotide fragment of the 5' UTR of HCV which includes the 9 most 3' nucleotides of the 5' UTR of HCV. Plasmid pHCV4-2 differs from plasmid pHCV2-1 in that it also contains the entire 5' UTR of HCV.

HCV specific sequences were amplified by Polymerase Chain Reaction (PCR) using pUC-T7-HCVTH (HCV Type 2) as a template and oligonucleotides comprises of the following sequences as PCR primers SEQ ID NO:16, SEQ ID NO:17 and SEQ ID NO:18.

The 5' PCR primers, SEQ ID NO:16 and SEQ ID NO:18 have NatI sites and the 3' PCR primer SEQ ID NO:17 has a SalI site. Three stop codons engineered into SEQ ID NO:17. SEQ ID NO:18 and SEQ ID NO:17 were used as primers to generate a PCR product which maps from position 1 to position 810 of the HCV genome and which contains the entire 5' HCV untranslated region and core coding region.

The use of SEQ ID NO:16 and SEQ ID NO:17 as primers resulted in a PCR product mapping from position 333 to position 810 of the HCV genome. This product contains the last 9 nucleotides of the HCV 5' untranslated region and the entire core coding region.

Both PCR products were digested with NatI and SalI (NED), gel purified and ligated into the NatI-SalI sites of plasmid Backbone A shown in Figure 2 to generate pHCV4-2 and pHCV2-1.

Plasmid backbone A is 3969 base pairs in length. It contains a PBR origin of replication for replicating in E. coli. It also contains a kanamycin resistance gene so that the plasmid can be selected in E. coli. Inserts such as the full-length HCV core gene, are cloned into a polylinker region which places the insert between and operably linked to the promoter and polyadenylation signal. Transcription of the cloned inserts is under the control of the CMV promoter and the RSV enhancer elements. A polyadenylation signal is provided by the presence of an SV40 poly A signal situated just 3' of the cloning site.

### Example 3: In vitro expression analysis

Expression of full-length HCV core from pHCV2-1 and pHCV4-2 was assayed in vitro in a rhabdomyosarcoma (RD) cell line. Cells were co-transfected with pHCV2-1 and a β-galactosidase reporter plasmid, pCMVB (Clonetech), pHCV4-2 and pCMVB, vector plasmid and pCMVB, or mock transfected. All analyses were conducted at 48 hours post-transfection. The transfection efficiency was determined by measuring the specific activity of β-galactosidase in cell lysates.

Transfected cells were analyzed by indirect immunofluorescence using either an anti-core monoclonal antibody or pooled HCV patent sera as the primary antibodies. FITC labelled anti-mouse lgG (Sigma) and FITC labelled antihuman IgG (Sigma) were used respectively as the secondary antibodies. Immunostaining was seen in cells that had been transfected with either pHCV2-1 or pHCV4-2 but not in cells transfected with the vector plasmid or mock transfected. Immunofluorescence in fixed cells appeared as punctate staining localized to the cytoplasm when either the monoclonal antibody or the pooled patient's sera was used as the source of primary antibody. There appeared to be no difference in the level or type of staining between pHCV2-1 and pHCV4-2 transfected cells. No staining was seen in unfixed cells indicating that the expressed core protein is probably not associated with the cell's surface membrane.

Transfected cell lysates were also analyzed by Western blot and immunoprecipitation. The presence or absence of the HCV 5' UTR seemed to have little effect upon the expression of core since pHCV2-1 and pHCV4-2 expressed equivalent amounts of core in the cell lines tested.

### Example 4: DNA Inoculation

Humoral immunity in a group of six mice was assessed following a single intramuscular inoculation of 100 µg of pHCV4-2 plus 0.25% bupivacaine. All mice were shown to have seroconverted 21 days after DNA inoculation. The humoral response persisted for several months. Studies are currently underway to evaluate the effect of multiple dosing.

This HCV core DNA-based vaccine expresses high levels of core antigen *in vitro* and induces a strong immune response *in vivo.*

## Claims

1. A recombinant nucleic acid molecule comprising a nucleotide coding sequence that encodes a fusion protein, said fusion protein comprising hepatitis B virus S gene product linked to amino acids 1-69 of hepatitis C virus core protein.

2. The recombinant nucleic acid molecule of claims 1, wherein said fusion protein comprises hepatitis B virus S gene product linked to amino acids 1-70 to 1-154 of hepatitis C virus core protein.

3. The recombinant nucleic acid molecule of claim 1, wherein said fusion protein comprises a fragment of hepatitis B virus pre S2 gene product linked to hepatitis B virus S gene product linked to amino acids 1-69 of hepatitis C virus core protein.

4. The recombinant nucleic acid molecule of claim 1, wherein said fusion protein comprises a fragment of hepatitis B virus pre S2 gene product linked to hepatitis B virus S gene product linked amino acids 1-70 to 1-154 of hepatitis C virus core protein.

5. The recombinant nucleic acid molecule of claim 1, wherein said fusion protein comprises a fragment of hepatitis B virus pre S1 gene product linked to a fragment of hepatitis B virus pre S2 gene product linked to hepatitis B virus S gene product linked to amino acids 1-69 of hepatitis C virus core protein.

6. The recombinant nucleic acid molecule of claim 1, wherein said fusion protein comprises a fragment of hepatitis B virus pre S1 gene product linked to a fragment of hepatitis B virus pre S2 gene product linked to hepatitis B virus S gene product linked to amino acids 1-70 to 1-154 of hepatitis C virus core protein.

7. The recombinant nucleic acid molecule of any one of the preceding claims wherein said nucleotide coding sequence is operably linked to cytomegalovirus promoter, Rous Sarcoma Virus enhancer, polyadenylation sequence, and optionally 5' UTR of hepatitis C virus.

8. A pharmaceutical composition comprising:
a) a recombinant nucleic acid molecule comprising a nucleotide coding sequence that encodes a fusion protein, said fusion protein comprising a hepatitis B virus S gene product linked to amino acids 1-69 of hepatitis C virus core protein, wherein said nucleotide coding sequence is operably linked to regulatory elements functional in human cells; and
b) a pharmaceutically acceptable carrier or diluent.

9. The pharmaceutical composition of claim 8, wherein said fusion protein comprises hepatitis B virus S gene product linked to amino acids 1-70 to 1-154 of hepatitis C virus core protein.

10. The pharmaceutical composition of claim 8, wherein said fusion protein comprises a fragment of hepatitis B virus pre S2 gene product linked to hepatitis B virus S gene product linked to amino acids 1-69 of hepatitis C virus core protein.

11. The pharmaceutical composition of claim 8, wherein said fusion protein comprises a fragment of hepatitis B virus pre S2 gene product linked to hepatitis B virus S gene product linked to amino acids 1-70 to 1-154 of hepatitis C virus core protein.

12. The pharmaceutical composition of claim 8, wherein said fusion protein comprises a fragment of hepatitis B virus pre S1 gene product linked to a fragment of hepatitis B virus pre S2 gene product linked to hepatitis B virus S gene product linked to amino acids 1-69 of hepatitis C virus core protein.

13. The pharmaceutical composition of claim 8 wherein said fusion protein comprises a fragment of hepatitis B virus pre S1 gene product linked to a fragment of hepatitis B virus pre S2 gene product linked to hepatitis B virus S gene product linked to amino acids 1-70 to 1-154 of hepatitis C virus core protein.

14. The pharmaceutical composition of any one of claims 8 to 13 wherein said regulatory elements functional in human cells comprise cytomegalovirus promoter, Rous Sarcoma Virus enhancer, - polyadenylation sequence, and optionally 5' UTR of hepatitis C virus.

15. An isolated and purified nucleic acid molecule having a nucleotide sequence comprising an incomplete hepatitis C viral genome, said genome comprising a coding sequence for a hepatitis C virus core protein and a 5' untranslated region wherein said 5' untranslated region is free of the 5' untranslated region of hepatitis C virus except the last 9 nucleotides of said 5' untranslated region.

16. A nucleic acid molecule according to claim 15 wherein said nucleic acid molecule is plasmid pHCV2-1.

17. An isolated and purified nucleic acid molecule consisting of a coding sequence encoding the hepatitis C virus core protein and one or more regulatory elements operably linked to said nucleotide sequence encoding the hepatitis C virus core protein.

18. A nucleic acid molecule according to any of claims 15 to 17 wherein said coding sequence comprises SEQ ID NO:15.

19. An isolated and purified nucleic acid molecule having a nucleotide sequence encoding SEQ ID NO:15.

20. A nucleic acid molecule according to any of claims 17 to 19 wherein said nucleotide sequence comprises at least the last 9 nucleotides of the 5' untranslated region of hepatitis C virus.

21. A nucleic acid molecule according to any of claims 17 to 19 wherein said nucleotide sequence comprises the entire 5' untranslated region of hepatitis C virus.

22. A nucleic acid molecule according to claim 19 comprising a promoter and polyadenylation sequence, wherein SEQ ID NO:15 is operably linked to said promoter and polyadenylation sequence.

23. A nucleic acid molecule according to any of claims 15 to 22 comprising a promoter and polyadenylation sequence, wherein said coding sequence is operably linked to said promoter and polyadenylation sequence.

24. A nucleic acid molecule according to any of claims 15 to 22 comprising an enhancer, wherein said coding sequence is operably linked to said enhancer.

25. A nucleic acid molecule according to any of claims 15 to 23 comprising a Kozak sequence, wherein said coding sequence is operably linked to said Kozak sequence.

26. A pharmaceutical composition comprising a nucleic acid molecule of any one of claims 15 to 25 and a pharmaceutically acceptable carrier or diluent.

## Patentansprüche

1. Rekombinantes Nucleinsäuremolekül, umfassend eine nucleotidkodierende Sequenz, die ein Fusionsprotein kodiert, wobei das Fusionsprotein ein Hepatitis-B-Virus-S-Genprodukt umfasst, das mit den Aminosäuren 1-69 des Hepatitis-C-Virus-Coreproteins verbunden ist.

2. Rekombinantes Nucleinsäuremolekül nach Anspruch 1, worin das Fusionsprotein ein Hepatitis-B-Virus-S-Genprodukt umfasst, das mit den Aminosäuren 1-70 bis 1-154 des Hepatitis-C-Virus-Coreproteins verbunden ist. ,

3. Rekombinantes Nucleinsäuremolekül nach Anspruch 1, worin das Fusionsprotein ein Fragment des Hepatitis-B-Virus-Prä-S2-Genprodukts umfasst, das mit einem Hepatitis-B-Virus-S-Genprodukt verbunden ist, das mit den Aminosäuren 1-69 des Hepatitis-C-Virus-Coreproteins verbunden ist.

4. Rekombinantes Nucleinsäuremolekül nach Anspruch 1, worin das Fusionsprotein ein Fragment des Hepatitis-B-Virus-Prä-S2-Genprodukts umfasst, das mit einem Hepatitis-B-Virus-S-Genprodukt verbunden ist, das mit den Aminosäuren 1-70 bis 1-154 des Hepatitis-C-Virus-Coreproteins verbunden ist.

5. Rekombinantes Nucleinsäuremolekül nach Anspruch 1, worin das Fusionsprotein ein Fragment des Hepatitis-B-Virus-Prä-S1-Genprodukts umfasst, das mit einem Hepatitis-B-Virus-Prä-S2-Genprodukt verbunden ist, das mit einem Hepatitis-B-Virus-S-Genprodukt verbunden ist, das mit den Aminosäuren 1-69 des Hepatitis-C-Virus-Coreproteins verbunden ist.

6. Rekombinantes Nucleinsäuremolekül nach Anspruch 1, worin das Fusionsprotein ein Fragment des Hepatitis-B-Virus-Prä-S1-Genprodukts umfasst, das mit einem Hepatitis-B-Virus-Prä-S2-Genprodukt verbunden ist, das mit einem Hepatitis-B-Virus-S-Genprodukt verbunden ist, das mit den Aminosäuren 1-70 bis 1-154 des Hepatitis-C-Virus-Coreproteins verbunden ist.

7. Rekombinantes Nucleinsäuremolekül nach einem der vorhergehenden Ansprüche, worin die nucleotidkodierende Sequenz operabel mit einem Cytomegalovirus-Promotor, einem Rous-Sarkom-Virus-Verstärker, einer Polyadenylierungssequenz und wahlweise: mit 5'-UTR des Hepatitis-C-Virus verbunden ist.

8. Pharmazeutische Zusammensetzung, umfassend:
a) ein rekombinantes Nucleinsäuremolekül, umfassend eine nucleotidkodierende Sequenz, die ein Fusionsprotein kodiert, wobei das Fusionsprotein ein Hepatitis-B-Virus-S-Genprodukt umfasst, das mit den Aminosäuren 1-69 des Hepatitis-C-Virus-Coreproteins verbunden ist, worin die nucleotidkodierende Sequenz operabel mit funktionellen Regulatorelementen in menschlichen Zellen verbunden ist; und
b) einen pharmazeutisch geeigneten Träger oder ein pharmazeutisch geeignetes Verdünnungsmittel.

9. Pharmazeutische Zusammensetzung nach Anspruch 8, worin das Fusionsprotein ein Hepatitis-B-Virus-S-Genprodukt umfasst, das mit den Aminosäuren 1-70 bis 1-154 des Hepatitis-C-Virus-Coreproteins verbunden ist.

10. Pharmazeutische Zusammensetzung nach Anspruch 8, worin das Fusionsprotein ein Fragment des Hepatitis-B-Virus-Prä-S2-Genprodukts umfasst, das mit einem Hepatitis-B-Virus-S-Genprodukt verbunden ist, das mit den Aminosäuren 1-69 des Hepatitis-C-Virus-Coreproteins verbunden ist.

11. Pharmazeutische Zusammensetzung nach Anspruch 8, worin das Fusionsprotein ein Fragment des Hepatitis-B-Virus-Prä-S2-Genprodukts umfasst, das mit einem Hepatitis-B-Virus-S-Genprodukt verbunden ist, das mit den Aminosäuren 1-70 bis 1-154 des Hepatitis-C-Virus-Coreproteins verbunden ist.

12. Pharmazeutische Zusammensetzung nach Anspruch 8, worin das Fusionsprotein ein Fragment des Hepatitis-B-Virus-Prä-S1-Genprodukts umfasst, das mit einem Hepatitis-B-Virus-Prä-S2-Genprodukt verbunden ist, das mit einem Hepatitis-B-Virus-S-Genprodukt verbunden ist, das mit den Aminosäuren 1-69 des Hepatitis-C-Virus-Coreproteins verbunden ist.

13. Pharmazeutische Zusammensetzung nach Anspruch 8, worin das Fusionsprotein ein Fragment des Hepatitis-B-Virus-Prä-S1-Genprodukts umfasst, das mit einem Hepatitis-B-Virus-Prä-S2-Genprodukt verbunden ist, das mit einem Hepatitis-B-Virus-S-Genprodukt verbunden ist, das mit den Aminosäuren 1-70 bis 1-154 des Hepatitis-C-Virus-Coreproteins verbunden ist.

14. Pharmazeutische Zusammensetzung nach einem der Ansprüche 8 bis 13, worin die funktionellen Regulatorelemente in menschlichen Zellen Cytomegalovirus-Promotor, Rous-Sarkom-Virus-Verstärker, Polyadenylierungssequenz und wahlweise 5'-UTR des Hepatitis-C-Virus umfassen.

15. Isoliertes und gereinigtes Nucleinsäuremolekül mit einer Nucleotidsequenz, die ein unvollständiges Hepatitis-C-virales Genom umfasst, wobei das Genom eine Codesequenz für ein Hepatitis-C-Virus-Coreprotein und eine 5'-untranslatierte Region umfasst, worin die 5'-untranslatierte Region frei ist von der 5'-untranslatierten Region des Hepatitis-C-Virus, mit Ausnahme der letzten 9 Nücleotide der 5'-untranslatierten Region.

16. Nucleinsäuremolekül nach Anspruch 15, worin das Nucleinsäuremolekül das Plasmid pHCV2-1 ist.

17. Isoliertes und gereinigtes Nucleinsäuremolekül, bestehend aus einer Codesequenz, die das Hepatitis-C-Virus-Coreprotein kodiert, und einem oder mehreren Regulatorelementen, die operabel mit der Nucleotidsequenz verbunden sind, die das Hepatitis-C-Virus-Coreprotein kodiert.

18. Nucleinsäuremolekül nach einem der Ansprüche 15 bis 17, worin die Codesequenz die Sequenzidentifizierungsnummer (SEQ ID Nr.) 15 hat.

19. Isoliertes und gereinigtes Nucleinsäuremelekül mit einer Nucleotidsequenz, die SEQ ID Nr. 15 kodiert.

20. Nucleinsäuremolekül nach einem der Ansprüche 17 bis 19, worin die Nucleotidsequenz mindestens die letzten 9 Nucleotide der 5'untranslatierten Region des Hepatitis-C-Virus umfasst.

21. Nucleinsäuremolekül nach einem der Ansprüche 17 bis 19, worin die Nucleotidsequenz die gesamte 5'-untranslatierte Region des Hepatitis-C-Virus umfasst.

22. Nucleinsäuremolekül nach Anspruch 19, umfassend eine Promotor- und Polyadenylierungssequenz, worin SEQ ID Nr. 15 operabel mit der Promotor- und Polyadenylierungssequenz verbunden ist.

23. Nucleinsäuremolekül nach einem der Ansprüche 15 bis 22, umfassend eine Promotor- und Polyadenylierungssequenz, worin die Codesequenz operabel :mit der Promotor- und Polyadenylierungssequenz verbunden ist.

24. Nucleinsäuremolekül nach einem der Ansprüche 15 bis 22, umfassend einen Verstärker, worin die Codesequenz operabel mit dem Verstärker verbunden ist.

25. Nucleinsäuremolekül nach einem der Ansprüche 15 bis 23, umfassend eine Kozak-Sequenz, worin die Codesequenz operabel mit der Kozak-Sequenz verbunden ist.

26. Pharmazeutische Zusammensetzung, umfassend ein Nucleinsäuremolekül aus einem der Ansprüche 15 bis 25 und einen pharmazeutisch geeigneten Träger oder ein pharmazeutisch geeignetes Verdünnungsmittel.

## Revendications

1. Molécule d'acide nucléique recombinante, comprenant une séquence codante nucléotidique qui code pour une protéine de fusion, ladite protéine de fusion comprenant le produit du gène S du virus de l'hépatite B lié aux acides aminés 1-69 de la protéine de noyau du virus de l'hépatite C.

2. Molécule d'acide nucléique recombinante selon la revendication 1, dans laquelle ladite protéine de fusion comprend le produit du gène S du virus de l'hépatite B lié aux acides aminés 1-70 à 1-154 de la protéine de noyau du virus de l'hépatite C.

3. Molécule d'acide nucléique recombinante selon la revendication 1, dans laquelle ladite protéine de fusion comprend un fragment du produit du gène pre S2 du virus de l'hépatite B lié au produit du gène S du virus de l'hépatite B lié aux acides aminés 1-69 de la protéine de noyau du virus de l'hépatite C.

4. Molécule d'acide nucléique recombinante selon la revendication 1, dans laquelle ladite protéine de fusion comprend un fragment du produit du gène pre S2 du virus de l'hépatite B lié au produit du gène S du virus de l'hépatite B lié aux acides aminés 1-70 à 1-154 de la protéine de noyau du virus de l'hépatite C.

5. Molécule d'acide nucléique recombinante selon la revendication 1, dans laquelle ladite protéine de fusion comprend un fragment du produit du gène pre S1 du virus de l'hépatite B lié à un fragment du produit du gène pre S2 du virus de l'hépatite B lié au produit du gène S du virus de l'hépatite B lié aux acides aminés 1-69 de la protéine de noyau du virus de l'hépatite C.

6. Molécule d'acide nucléique recombinante selon la revendication 1, dans laquelle ladite protéine de fusion comprend un fragment du produit du gène pre S1 du virus de l'hépatite B lié à un fragment du produit du gène pre S2 du virus de l'hépatite B lié au produit du gène S du virus de l'hépatite B lié aux acides aminés 1-70 à 1-154 de la protéine de noyau du virus de l'hépatite C.

7. Molécule d'acide nucléique recombinante selon l'une quelconque des revendications précédentes, dans laquelle ladite séquence codante nucléotidique est liée fonctionnellement au promoteur du cytomégalovirus, à l'activateur du virus du Sarcome de Rous, à une séquence de polyadénylation et facultativement à la région 5' UTR du virus de l'hépatite C.

8. Composition pharmaceutique comprenant :
(a) une molécule d'acide nucléique recombinante, comprenant une séquence codante nucléotidique qui code pour une protéine de fusion, ladite protéine de fusion comprenant un produit du gène S du virus de l'hépatite B lié aux acides aminés 1-69 de la protéine de noyau du virus de l'hépatite C, ladite séquence codante nucléotidique étant liée de façon fonctionnelle aux éléments régulateurs fonctionnels dans des cellules humaines ; et
(b) un support ou diluant pharmaceutiquement acceptable.

9. Composition pharmaceutique selon la revendication 8, dans laquelle ladite protéine de fusion comprend le produit du gène S du virus de l'hépatite B lié aux acides aminés 1-70 à 1-154 de la protéine de noyau du virus de l'hépatite C.

10. Composition pharmaceutique selon la revendication 8, dans laquelle ladite protéine de fusion comprend un fragment du produit du gène pre S2 du virus de l'hépatite B lié au produit du gène S du virus de l'hépatite B lié aux acides aminés 1-69 de la protéine de noyau du virus de l'hépatite C.

11. Composition pharmaceutique selon la revendication 8, dans laquelle ladite protéine de fusion comprend un fragment du produit du gène pre S2 du virus de l'hépatite B lié au produit du gène S du virus de l'hépatite B lié aux acides aminés 1-70 à 1-154 de la protéine de noyau du virus de l'hépatite C.

12. Composition pharmaceutique selon la revendication 8, dans laquelle ladite protéine de fusion comprend un fragment du produit du gène pre S1 du virus de l'hépatite B lié à un fragment du produit du gène pre S2 du virus de l'hépatite B lié au produit du gène S du virus de l'hépatite B lié aux acides aminés 1-69 de la protéine de noyau du virus de l'hépatite C.

13. Composition pharmaceutique selon la revendication 8, dans laquelle ladite protéine de fusion comprend un fragment du produit du gène pre S1 du virus de l'hépatite B lié à un fragment du produit du gène pre S2 du virus de l'hépatite B lié au produit du gène S du virus de l'hépatite B lié aux acides aminés 1-70 à 1-154 de la protéine de noyau du virus de l'hépatite C.

14. Composition pharmaceutique selon l'une quelconque des revendications 8 à 13, dans laquelle lesdits éléments régulateurs fonctionnels dans des cellules humaines comprennent le promoteur du cytomégalovirus, l'activateur du Virus du Sarcome de Rous, une séquence de polyadénylation, et facultativement la région 5' UTR du virus de l'hépatite C.

15. Molécule d'acide nucléique isolée et purifiée ayant une séquence nucléotidique comprenant un génome viral de l'hépatite C incomplet, ledit génome comprenant une séquence codante pour une protéine de noyau du virus de l'hépatite C et une région 5' non traduite, ladite région 5' non traduite étant exempte de la région 5' non traduite du virus de l'hépatite C excepté les 9 derniers nucléotides de ladite région 5' non traduite.

16. Molécule d'acide nucléique selon la revendication 15, dans laquelle ladite molécule d'acide nucléique est le plasmide pHCV2-1.

17. Molécule d'acide nucléique isolée et purifiée consistant en une séquence codante codant pour la protéine de noyau du virus de l'hépatite C et un ou plusieurs éléments régulateurs liés de façon fonctionnelle à ladite séquence nucléotidique codant pour la protéine de noyau du virus de l'hépatite C.

18. Molécule d'acide nucléique selon l'une quelconque des revendications 15 à 17, dans laquelle ladite séquence codante comprend SEQ ID NO:15.

19. Molécule d'acide nucléique isolée et purifiée ayant une séquence nucléotidique codant pour SEQ ID NO:15.

20. Molécule d'acide nucléique selon l'une quelconque des revendications 17 à 19, dans laquelle ladite séquence nucléotidique comprend au moins les 9 derniers nucléotides de la région 5' non traduite du virus de l'hépatite C.

21. Molécule d'acide nucléique selon l'une quelconque des revendications 17 à 19, dans laquelle ladite séquence nucléotidique comprend la région 5' non traduite entière du virus de l'hépatite C.

22. Molécule d'acide nucléique selon la revendication 19, comprenant un promoteur et une séquence de polyadénylation, SEQ ID NO:15 étant liée de façon fonctionnelle audit promoteur et à la séquence de polyadénylation.

23. Molécule d'acide nucléique selon l'une quelconque des revendications 15 à 22, comprenant un promoteur et une séquence de polyadénylation, ladite séquence codante étant liée de façon fonctionnelle audit promoteur et à ladite séquence de polyadénylation.

24. Molécule d'acide nucléique selon l'une quelconque des revendications 15 à 22, comprenant un activateur, ladite séquence codante étant liée de façon fonctionnelle audit activateur.

25. Molécule d'acide nucléique selon l'une quelconque des revendications 15 à 23, comprenant une séquence Kozak, ladite séquence codante étant liée de façon fonctionnelle à ladite séquence Kozak.

26. Composition pharmaceutique comprenant une molécule d'acide nucléique telle que définie à l'une quelconque des revendications 15 à 25 et un support ou diluant pharmaceutiquement acceptable.


### SEQUENCE LISTING

(1) GENERAL INFORMATION:
   (i) APPLICANT: Wands, Jack
      Tokushige, Katsutoshi
      Wakita, Takaji
      Pachuk, Catherine J.
      Zurawski, Jr., Vincent R.
      Coney, Leslie R.
   (ii) TITLE OF INVENTION: HEPATITIS VIRUS VACCINES
   (iii) NUMBER OF SEQUENCES: 18
   (iv) CORRESPONDENCE ADDRESS:
      (A) ADDRESSEE: Woodcock, Washburn, Kurtz, Mackiewicz & Norris
      (B) STREET: One Liberty Place, 46th floor
      (C) CITY: Philadelphia
      (D) STATE: PA
      (E) COUNTRY: USA
      (F) ZIP: 19103
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: WordPerfect 5.1
   (vi) CURRENT APPLICATION DATA:
      (A) APPLICATION NUMBER: N/A
      (B) FILING DATE: Herewith
      (C) CLASSIFICATION:
   (vii) PRIOR APPLICATION DATA:
      (A) APPLICATION NUMBER: US 08/318,248
      (B) FILING DATE: 05-OCT-1994
      (C) CLASSIFICATION:
   (vii) PRIOR APPLICATION DATA:
      (A) APPLICATION NUMBER: US 08/467,859
      (B) FILING DATE: 06-JUN-1995
      (C) CLASSIFICATION:
   (viii) ATTORNEY/AGENT INFORMATION:
      (A) NAME: DeLuca, Mark
      (B) REGISTRATION NUMBER: 33,229
      (C) REFERENCE/DOCKET NUMBER: APOL-0238
   (ix) TELECOMMUNICATION INFORMATION:
      (A) TELEPHONE: (215) 568-3100
      (B) TELEFAX: (215) 568-3439
(2) INFORMATION FOR SEQ ID NO:1:
   (i) SEQUENCE CHARACTERISTICS:
      (A) LENGTH: 462 base pairs
      (B) TYPE: nucleic acid
      (C) STRANDEDNESS: double
      (D) TOPOLOGY: linear
   (xi) SEQUENCE DESCRIPTION: SEQ ID NO:1:
   ATG AGC ACG AAT CCT AAA CCT CAA AGA AAA ACC AAA CGT AAC ACC AAC  48
   Met Ser Thr Asn Pro Lys Pro Gln Arg Lys Thr Lys Arg Asn Thr Asn
    1               5                  10                  15
   CGC CGC CCA CAG GAC GTC AAG TTC CCG GGC GGT GGT CAG ATC GTT GGT  96
   Arg Arg Pro Gln Asp Val Lys Phe Pro Gly Gly Gly Gln Ile Val Gly
                20                  25                  30
   GGA GTT TAC CTG TTG CCG CGC AGG GGC CCC AGG TTG GGT GTG CGC GCG 144
   Gly Val Tyr Leu Leu Pro Arg Arg Gly Pro Arg Leu Gly Val Arg Ala
           35                  40                  45
   ACT AGG AAG ACT TCC GAG CGG TCG CAA CCT CGT GGA AGG CGA CAA CCT 192
   Thr Arg Lys Thr Ser Glu Arg Ser Gln Pro Arg Gly Arg Arg Gln Pro
       50                  55                  60
   ATC CCC AAG GAT CGC CGG CCC GAG GGC AGG GCC TGG GCT CAA CCT GGG 240
   Ile Pro Lys Asp Arg Arg Pro Glu Gly Arg Ala Trp Ala Gln Pro Gly
   65                  70                  75                  80
   TAC CCT TGG CCC CTC TAT GGC AAC GAG GGC ATG GGG TGG GCA GGA TGG 288
   Tyr Pro Trp Pro Leu Tyr Gly Asn Glu Gly Met Gly Trp Ala Gly Trp
                   85                  90                  95
   CTC CTG TCA CCC CGT GGC TCC CGG CCT AGT TGG GGC CCC AAT GAC CCC 336
   Leu Leu Ser Pro Arg Gly Ser Arg Pro Ser Trp Gly Pro Asn Asp Pro
               100                 105                 110
   CGG CGT AGG TCG CGT AAT TTG GGT AAA GTC ATC GAT ACC CTT ACA TGC 384
   Arg Arg Arg Ser Arg Asn Leu Gly Lys Val Ile Asp Thr Leu Thr Cys
           115                 120                 125
   GGC TTC GCC GAC CTC ATG GGG TAC ATT CCG CTC GTC GGC GCT CCC TTG 432
   Gly Phe Ala Asp Leu Met Gly Tyr Ile Pro Leu Val Gly Ala Pro Leu
       130                 135                 140
   GGG GGC GCT GCC AGG GCC TTG GCG CAT GGC 462
   Gly Gly Ala Ala Arg Ala Leu Ala His Gly
   145                 150
(2) INFORMATION FOR SEQ ID NO:2:
   (i) SEQUENCE CHARACTERISTICS:
      (A) LENGTH: 154 amino-acids
      (B) TYPE: amino acid
      (D) TOPOLOGY: linear
   (xi) SEQUENCE DESCRIPTION: SEQ ID NO:2:
   Met Ser Thr Asn Pro Lys Pro Gln Arg Lys Thr Lys Arg Asn Thr Asn
    1               5                  10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Pro Glu Gly Arg Ala Trp Ala Gln Pro Gly
   65                  70                  75                  80
   Tyr Pro Trp Pro Leu Tyr Gly Asn Glu Gly Met Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly
   145                 150
(2) INFORMATION FOR SEQ ID NO:3:
   (i) SEQUENCE CHARACTERISTICS:
      (A) LENGTH: 9 base pairs
      (B) TYPE: nucleic acid
      (C) STRANDEDNESS: double
      (D) TOPOLOGY: linear
   (xi) SEQUENCE DESCRIPTION: SEQ ID NO:3:
   CCGTGCACC 9
(2) INFORMATION.FOR SEQ ID NO:4:
   (i) SEQUENCE CHARACTERISTICS:
      (A) LENGTH: 341 base pairs
      (B) TYPE: nucleic acid
      (C) STRANDEDNESS: double
      (D) TOPOLOGY: linear
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 1200 base pairs
      (B) TYPE: nucleic acid
      (C) STRANDEDNESS: double
      (D) TOPOLOGY: linear
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 21 bases
      (B) TYPE: nucleic acid
      (C) STRANDEDNESS: single
      (D) TOPOLOGY: linear
   (xi) SEQUENCE DESCRIPTION: SEQ ID NO:6:
   GGGTCACCAT ATTCTTGGGA A   21
(2) INFORMATION FOR SEQ ID NO:7:
   (i) SEQUENCE CHARACTERISTICS:
      (A) LENGTH: 21 bases
      (B) TYPE: nucleic acid
      (C) STRANDEDNESS: single
      (D) TOPOLOGY: linear
   (xi) SEQUENCE DESCRIPTION: SEQ ID NO:7:
   GCAGCAAAGC CCAAAAGACC C   21
(2) INFORMATION FOR SEQ ID NO:8:
   (i) SEQUENCE CHARACTERISTICS:
      (A) LENGTH: 28 bases
      (B) TYPE: nucleic acid
      (C) STRANDEDNESS: single
      (D) TOPOLOGY: linear
   (xi) SEQUENCE DESCRIPTION: SEQ ID NO:8:
   CGGGATCCAT GAGCACGAAT CCTAAACC   28
(2) INFORMATION FOR SEQ ID NO:9:
   (i) SEQUENCE CHARACTERISTICS:
      (A) LENGTH: 34 bases
      (B) TYPE: nucleic acid
      (C) STRANDEDNESS: single
      (D) TOPOLOGY: linear
   (xi) SEQUENCE DESCRIPTION: SEQ ID NO:9:
   TCTCTAGATT ACTAGCCATG CGCCAAGGCC CTGG 34
(2) INFORMATION FOR SEQ ID NO:10:
   (i) SEQUENCE CHARACTERISTICS:
      (A) LENGTH: 27 bases
      (B) TYPE: nucleic acid
      (C) STRANDEDNESS: single
      (D) TOPOLOGY: linear
   (xi) SEQUENCE DESCRIPTION: SEQ ID NO:10:
   CATGGATCCA ATGTATACCC AAAGACA 27
(2) INFORMATION FOR SEQ ID NO:11:
   (i) SEQUENCE CHARACTERISTICS:
      (A) LENGTH: 34 bases
      (B) TYPE: nucleic acid
      (C) STRANDEDNESS: single
      (D) TOPOLOGY: linear
   (xi) SEQUENCE DESCRIPTION: SEQ ID NO:11:
   AGACACAAGC TTATGGGAGG TTGGTCTTCC AAAC 34
(2) INFORMATION FOR SEQ ID NO:12:
   (i) SEQUENCE CHARACTERISTICS:
      (A) LENGTH: 31 bases
      (B) TYPE: nucleic acid
      (C) STRANDEDNESS: single
      (D) TOPOLOGY: linear
   (xi) SEQUENCE DESCRIPTION: SEQ ID NO:12:
   AGACACAAGC TTGCCGCCAT GCAGTGGAAC T
(2) INFORMATION FOR SEQ ID NO:13:
   (i) SEQUENCE CHARACTERISTICS:
      (A) LENGTH: 9 base pairs
      (B) TYPE: nucleic acid
      (C) STRANDEDNESS: double
      (D) TOPOLOGY: linear
   (xi) SEQUENCE DESCRIPTION: SEQ ID NO:13:
   GCCGCCATG 9
(2) INFORMATION FOR SEQ ID NO:14:
   (i) SEQUENCE CHARACTERISTICS;
      (A) LENGTH: 923 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: CDS
      (B) LOCATION: 342..914
   (xi) SEQUENCE DESCRIPTION: SEQ ID NO:14:
   GCCAGCCCCC GATTGGGGGC GACACTCCAC CATAGATCAC TCCCCTGTGA GGAACTACTG      60
   TCTTCACGCA GAAAGCGTCT AGCCATGGCG TTAGTATGAG TGTCGTGCAG CCTCCAGGAC     120
   CCCCCCTCCC GGGAGAGCCA TAGTGGTCTG CGGAACCGGT GAGTACACCG GAATTGCCAG     180
   GACGACCGGG TCCTTTCTTG GATCAACCCG CTCAATGCCT GGAGATTTGG GCGTGCCCCC     240
   GCGAGACTGC TAGCCGAGTA GTGTTGGGTC GCGAAAGGCC TTGTGGTACT GCCTGATAGG     300
   GTGCTTGCGA GTGCCCCGGG AGGTCTCGTA GACCGTGCAC C ATG AGC ACG AAT         353
                                                 Met Ser Thr Asn
                                                   1
   CCT AAA CCT CAA AGA AAA ACC AAA CGT AAC ACC AAC CGC CGC CCA CAG       401
   Pro Lys Pro Gln Arg Lys Thr Lys Arg Asn Thr Asn Arg Arg Pro Gln
     5                  10                  15                  20
   GAC CTC AAG TTC CCG GGC GGT GGT CAG ATC GTT GGT GGA GTT TAC CTG       449
   Asp Leu Lys Phe Pro Gly Gly Gly Gln Ile Val Gly Gly Val Tyr Leu
                    25                  30                  35
   TTG CCG CGC AGG GGC CCC AGG TTG GGT GTG CGC GCG ACT AGG AAG ACT       497
   Leu Pro Arg Arg Gly Pro Arg Leu Gly Val Arg Ala Thr Arg Lys Thr
                40                  45                  50
   TCC GAG CGG TCG CAA CCT CGT GGA AGG CGA CAA CCT ATC CCC AAG GAT       545
   Ser Glu Arg Ser Gln Pro Arg Gly Arg Arg Gln Pro Ile Pro Lys Asp
            55                  60                  65
   CGC CGG CCC GAG GGC AGG GCC TGG GCT CAA CCT GGG TAC CCT TGG CCC       593
   Arg Arg Pro Glu Gly Arg Ala Trp Ala Gln Pro Gly Tyr Pro Trp Pro
        70                  75                  80
   CTC TAT GGC AAC GAG GGC ATG GGG TGG GCA GGA TGG CTC CTG TCA CCC       641
   Leu Tyr Gly Asn Glu Gly Met Gly Trp Ala Gly Trp Leu Leu Ser Pro
    85                  90                  95                 100
   CGT GGC TCC CGG CCT AGT TGG GGC CCC AAT GAC CCC CGG CGT AGG TCG       689
   Arg Gly Ser Arg Pro Ser Trp Gly Pro Asn Asp Pro Arg Arg Arg Ser
                   105                 110                 115
   CGT AAT TTG GGT AAA GTC ATC GAT ACC CTT ACA TGC GGC TTC GCC GAC       737
   Arg Asn Leu Gly Lys Val Ile Asp Thr Leu Thr Cys Gly Phe Ala Asp
               120                 125                 130
   CTC ATG GGG TAC ATT CCG CTC GTC GGC GCT CCC ATG GGG GGC GCT GCC       785
   Leu Met Gly Tyr Ile Pro Leu Val Gly Ala Pro Met Gly Gly Ala Ala
           135                 140                 145
   AGG GCC TTG GCG CAT GGC GTC CGG GTT CTG GAG GAC GGC GTG AAC TAT       833
   Arg Ala Leu Ala His Gly Val Arg Val Leu Glu Asp Gly Val Asn Tyr
       150                 155                 160
   GCA ACA GGG AAT CTG CCC GGT TGC TCT TTC TCT ATC TTC CTC TTG GCT       881
   Ala Thr Gly Asn Leu Pro Gly Cys Ser Phe Ser Ile Phe Leu Leu Ala
   165                 170                 175                 180
   CTG CTG TCC TGT TTG ACC ATC CCA GCT TCC GCT TAATAATAA                 923
   Leu Leu Ser Cys Leu Thr Ile Pro Ala Ser Ala
                   185                 190
(2) INFORMATION FOR SEQ ID NO:15:
   (i) SEQUENCE CHARACTERISTICS:
      (A) LENGTH: 191 amino acids
      (B) TYPE: amino acid
      (D) TOPOLOGY: linear
   (ii) MOLECULE TYPE: protein
   (xi) SEQUENCE DESCRIPTION: SEQ ID NO:15:
   Met Ser Thr Asn Pro Lys Pro Gln Arg Lys Thr Lys Arg Asn Thr Asn
    1               5                  10                  15
   Arg Arg Pro Gln Asp Leu Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Pro Glu Gly Arg Ala Trp Ala Gln Pro Gly
   65                  70                  75                  80
   Tyr Pro Trp Pro Leu Tyr Gly Asn Glu Gly Met Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Met
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala
               180                 185                 190
(2) INFORMATION FOR SEQ ID NO:16:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:16:
   AAAAAGGGAA GGAAGGCGGC CGCCCGTGCA CCATGAGCAC GAATCCT     47
(2) INFORMATION FOR SEQ ID NO:17:
   (i) SEQUENCE CHARACTERISTICS:
      (A) LENGTH: 57 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:17:
   AAAAAGGGAA GGAAGGTCGA CTTATTATTA TTAAGCGGAA GCTGGGATGG TCAAACA 57
(2) INFORMATION FOR SEQ ID NO 18:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:18:
   AAAAAGGGAA GGAAGGCGGC CGCGCCAGCC CCCGATTGGG GGCGACA 47